# EUROPEAN PATENT SPECIFICATION

(11) **EP 0 586 397 B1**
(45) Date of publication and mention of the grant of the patent: **02.12.1998**
(21) Application number: 92908570.2
(22) Date of filing: 27.02.1992
(51) Int. Cl.: C12Q 1/26, C12Q 1/00, C12Q 1/60, C12Q 1/54

(54) **IMPROVED METHOD AND REAGENT FOR DETERMINATION OF AN ANALYTE**
VERBESSERTES VERFAHREN UND MITTEL ZUR BESTIMMUNG EINES ANALYTEN
PROCEDE ET REACTIF AMELIORES POUR LA DETERMINATION D'UN ANALYTE

(30) Priority: 27.02.1991 US 661728
(43) Date of publication of application: 16.03.1994
(73) Proprietor: BOEHRINGER MANNHEIM CORPORATION, Indianapolis, Indiana 46250-0528 (US)
(72) Inventor: KOST, Kent, M., Fishers, IN 46038 (US)
(74) Representative: Jung, Michael, Dr.
(86) International application number: US9201659
(87) International publication number: WO9215704

(56) References cited:
- EP-A- 0 290 223
- US-A- 4 576 913
- US-A- 4 929 545
- Römpp's Chemie-Lexikon, Vol. 5, 8th ed. (1987), 3530 - 3531

## Description

### FIELD OF THE INVENTION

This invention relates generally to a method for determining an analyte in a sample and to a reagent composition useful in the method. More specifically, this invention relates to a reagent having improved stability and also to a reagent having an increased dynamic range of change in response characteristics or response curve.

### BACKGROUND AND PRIOR ART

The central concern of clinical chemistry is the qualitive and quantitative determination of specific analytes in samples. Of special concern is the analysis of body fluid samples, such as blood, serum, urine and so forth. Determination of the presence and/or amount of various analytes, followed by comparison to established parameters determines diagnosis of diseased or abnormal states.

The literature on analytical determination of body fluid samples is large, as the art has investigated the determination of, e.g., glucose, cholesterol, creatine, sarcosine, urea and other substances in samples of blood, serum, urine and so forth.

There are many types of assays for making such analytical determinations. One type of assay is based on a sequence of reactions. The first reaction is between an analyte and an enzyme which is specific for that analyte. In that reaction the enzyme "oxidizes" the analyte, causing it to lose an electron. In a subsequent reaction the electron is accepted by a mediator which is thus reduced. The reduced mediator can then be used in a variety of indicator systems to determine the presence and concentration of the analyte.

One such indicator system is described in commonly asigned US Patent No. 4,929,545. In that system a ferricyanide is reduced to a ferrocyanide by the electron lost by the analyte. The ferrocyanide combines with a ferric compound to form a color. The presence and degree of color change are indications of the presence and concentration, respectively, of the analyte in a specimen.

The degree of color change can be measured by a reflectance photometer. The photometer is normally a part of a meter which uses an internal "look-up" table or an algorhythm to convert the change in reflectance-to analyte concentration.

Other similar systems use ferrocene or one of its analogs as a mediator.

Another system for using the reduced mediator to determine the presence or concentration of an analyte is described in commonly assigned copending U.S. patent application USSN 07/451,671, filed December 15, 1989, corresponding to EP 0 505 494 B1. In that system the reduced mediator is re-oxidized by the application of a current, and the decay in the current after a specific time is measured and used to determine the concentration of the analyte.

In either system increased stability of the reagent containing the mediator is always desirable. Stability is necessary to obtain a commercially interesting shelf life. In the system described in US Patent No. 4,929,545, as in any system where change in light reflectance is measured over a range, it is desirable to increase the range. A larger range increases the size of the increments of measured reflectance and, thus, increases accuracy.

### SUMMARY OF THE INVENTION

It is an object of the present invention to increase the stability of of a reagent containing a reducable mediator.

It is also an object of this invention to increase the range of change in light reflectance of a reagent containing a reducable mediator and a component which changes color in response to reduction of the mediator.

These and other objects are accomplished by a reagent composition which comprises a reducible mediator and an oxidizing agent which will' improve the stability of the reagent prior to use in an assay and which will not significantly interfere with the reduction of the mediator during performance of the assay.
In particular, further objects of this invention are:
- A reagent composition suitable for use in color change assays for the determination of an analyte in a liquid sample and having an increased dynamic range of change in reflectance and/or absorbance comprising an enzyme which will act on analyte present in the sample, a soluble ferricyanide mediator compound which will be reduced in response to the action of the enzyme on the analyte to effect a color change in the reagent corresponding to the presence or concentration of the analyte and from about 0.9 x 10-4 to about 17.5 x 10-4 grams per kU of enzyme of an alkali metal dichromate which is soluble in the reagent composition.
- Method for improving the dynamic range of light absorbance of a composition useful for the determination of the presence or concentration of an analyte in a liquid sample, the composition comprising an enzyme, a soluble ferricyanide mediator which is reducible in response to a reaction between an analyte and the enzyme and a reactant which acts with the reduced form of the mediator to change the light absorbance of the composition over a ranqe, the method comprising adding an alkali metal dichromate to the composition in a concentration of from about 0.9 x 10-4 to about 17.5 x 10-4 grams per kU of enzyme, whereby the dynamic range over which the change in light absorbance occurs is increased.

The dependent claims set out particular embodiments of the invention.

This invention is based on the surprising and unexpected discoveries that such an oxidizing agent can be selected and that the selected oxidizing agent increases the range of change in reflectance of an ingredient which changes color in response to reduction of the mediator.

Any oxidizing agent which will inhibit premature reduction of the reducible mediator but which will not compete successfully with the reduction of the mediator during performance of an assay is useful in the invention. In the color-based assay described in US Patent No. 4,929,545, when the mediator is a ferricyanide, the indicator is a soluble ferric compound such as ferric chloride or ferric sulphate and the enzyme is glucose oxidase or cholesterol oxidase, it has been discovered that an alkali metal dichromate, such as sodium or potassium dichromate is suitable for use in the invention.

A preferred reagent for use in such color-based assays for glucose in blood includes potassium ferricyanide, a soluble ferric compound, glucose oxidase and potassium dichromate in a concentration of from a trace to about 17.5 x 10⁻⁴ grams per kU of glucose oxidase. Such a reagent will have a stability many times greater than the same reagent without the dichromate and will have an increased dynamic range of change in the reflectance region of interest.

The operation of the oxidizing agent with regard to improved stability is not completely understood, although it is believed that the oxidizing agent oxidizes impurities in the other reagent components which might cause premature color change in the indicator. It is also believed that the oxidizing agent stabilizes the mediator with regard to the reducing effects of light, heat and humidity but not with regard to the reducing effects of the electron provided by the reaction between the analyte and the enzyme, surprisingly all without inactivating the enzyme.

The operation of the oxidizing agent to increase the dynamic range of the reagent dose response curve is not fully understood at the present time. Although it is an observed effect, it would be expected that, in the preferred embodiment for example, the oxidizing agent would limit the range of color change because it would act on the reduced ferrocyanide to suppress the reaction between the ferrocyanide and the ferric compound to product Prussian blue.

It has been found through experimentation that trace quantities of a dichromate will improve the stability of the reagent, that quantities of 1.3 x 10⁻⁴ grams per kU of enzyme will increase stability about twelve-fold at elevated temperatures, and that amounts up to 17.5 x 10⁻⁴ grams per kU of enzyme can be used without hindering color development.

It has also been found that quantities of dichromate as small as 0.9 x 10⁻⁴ grams per kU of enzyme will increase the dynamic range of the reflectance region of interest by about five percentage points, which is an increase of about 10% in the size of the range.

The reagent composition of the present invention has useful commercial application in assays for components of fluid samples. A preferred use is in an assay device such as is described in a copending, commonly assigned patent application filed on even date herewith as USSN 07/661,788, filed February 27, 1991, by Ralph Mccroskey et al., now US Patent No. 5,272,895, where the reagent is coated on a support in a novel teststrip architecture. In that embodiment the reagent layer is contacted by whole blood which is held against the reagent layer in a predetermined volume.

### DETAILED DESCRIPTION AND EXAMPLES

The preferred embodiments of the invention are illustrated in the following examples which are intended to be illuminative, but not limiting.

### EXAMPLE 1

Test strips were prepared and used in order to show the use of the invention in reflectance assays.

A coating mass was prepared which contained, per kilogram of the coating mass, 30 g. 4-amino butyric acid, 3.9 g. of ferric sulfate, 36 g. of ferricyanide salt and 1,000 kU of glucose oxidase. Nonreactive ingredients which made up the remainder of the mass included TiO₂ as a white pigment, TWEEN®-20 (nonionic surfactant), PROPIOFAN®-70D (film former) and NATROSOL® (thickening agent). The pH of this mass was 4.1.

Potassium dichromate was added to aliquots of the coating mass in concentrations ranging from a trace to 17.52 x 10⁻⁴ grams as shown in Tables 1a and 1b, below. Some of the aliquots were then subjected to accelerated degradation testing in a thermal stress chamber. The accelerated testing subjects the reagent to elevated temperatures in order to predict a normal shelf life.

After stressing, the aliquots were examined for stability. Color change in the reagent before contact with a fluid sample containing an analyte specific for the enzyme indicates instability and diminished shelf life. Concentrations of dichromate in the various aliquots, the duration of the test, the elevated temperature of the stress chamber and the resulting increase in shelf life over the aliquoit with no dichromate is shown in Table la, below. The observations indicate that the addition of a dichromate in even trace amounts can improve significantly, while greater amounts will improve stability for longer times.

Other aliquots were subjected to samples of a glucose-containing control solution which contained glucose in the same range as normal human blood. Table 1b shows the dichromate concentrations of the various aliquots and indicates whether color development is acceptable or hindered. The results indicate that the dichromate does not hinder color development of the reagent until relatively high concentrations are reached.

**TABLE 1a**

| Dichromate Concentration (grams/kU of enzyme) | Stability (normalized) |
|---|---|
| 0 | x @ 25° C. |
| | y @ 35° C. |
| | |
| trace | 3x |
| | 3y |
| | |
| 0.4 x 10⁻⁴ | 5x |
| | 8y |
| | |
| 1.3 x 10⁻⁴ | 6x |
| | 12y |

**TABLE 1b**

| Dichromate grams/ kU enzyme | Color Performance |
|---|---|
| 4.83 x 10⁻⁴ | Acceptable |
| | |
| 8.76 x 10⁻⁴ | Acceptable |
| | |
| 13.14 x 10⁻⁴ | Acceptable |
| | |
| 17.14 x 10⁻⁴ | Color Development Slowed. |

This experiment shows that the addition of a soluble dichromate to a reagent containing a mediator, an enzyme specific for an analyte and an indicator which reacts with the reduced form of the mediator to produce a color will improve the stability of the reagent without inactivating the enzyme and without supressing color development over a useful range of concentrations.

### EXAMPLE 2

Teststrips were prepared and used in order to show the improvement in the range of change in reflectance when the dichromate oxidizer is added to the reagent.

Two batches of test strip reagent is prepared as in Example 1. One batch contains no dichromate, and the other batch contains about 0.9 x 10⁻⁴ grams of dichromate per kU of enzyme. Each batch is divided into aliquots and coated onto a transparent carrier layers which are part of test strips. Test strips made with each batch of reagent are subjected to samples of a glucose solution containing differing concentrations of glucose. The layers were then interrogated from the side opposite the coated side by a reflectance photometer. The ranges of reflectance of light at 660 nM for reagent containing the dichromate and for reagent not containing the dichromate are shown In Table 2, below.

**TABLE 2**

| Reagent | % Reflectance at 0 mg/dl glucose | % Reflectance at 500 mg/dl glucose |
|---|---|---|
| Without dichromate | 53 | 18 |
| With dichromate | 58 | 18 |

This experiment shows that the addition of the dichromate to the reagent increases the dynamic range of change in reflectance by five percentage points, which is an increase of about 10 percent in the size of the range.

## Claims

1. A reagent composition suitable for use in color change assays for the determination of an analyte in a liquid sample and having an increased dynamic range of change in light reflectance and/or absorbance, comprising an enzyme which will act on analyte present in the sample, a soluble ferricyanide mediator compound which will be reduced in response to the action of the enzyme on the analyte to effect a color change in the reagent corresponding to the presence or concentration of the analyte and from about 0.9 x 10-4 to about 17.5 x 10-4 grams per kU of enzyme of an alkali metal dichromate which is soluble in the reagent composition.

2. The reagent composition of Claim 1 wherein the enzyme is glucose oxidase or cholseterol oxidase.

3. The reagent composition of Claim 1 wherein the enzyme is glucose oxidase and the alkali metal dichromate is sodium dichromate or potasium dichromate.

4. The reagent composition of Claim 1, wherein the soluble ferricyanide compound is reducible in the presence of the reaction between the enzyme and the analyte to produce a ferrocyanide compound, further comprising a soluble ferric compound which reacts with a ferrocyanide compound to form a visually or optically detectable reaction product therebetween which reaction product changes the light absorbance of the reagent composition relative to the concentration of the analyte, and a buffer which does not prevent formation of the reaction product, wherein the compositiion has a pH of from about 3.0 to about 7.0.

5. The composition of Claim 4 wherein the alkali metal dichromate is sodium dichromate or potasium dichromate.

6. The composition of Claim 5 wherein the enzyme is glucose oxidase or cholseterol oxidase.

7. The composition of Claim 6 wherein the enzyme is glucose oxidase and the alkali metal dichromate is potasium dichromate.

8. The composition of Claims 1 or 4 coated onto a carrier.

9. Method for improving the dynamic range of light absorbance of a composition useful for the determination of the presence or the concentration of an analyte in a liquid sample, the composition comprising an enzyme, a soluble ferricyanide mediator which is reducible in response to a reaction between an analyte and the enzyme and a reactant which acts with the reduced form of the mediator to change the light absorbance of the composition over a range, the method comprising adding an alkali metal dichromate to the composition in a concentration of from about 0.9 x 10-4 to about 17.5 x 10-4 grams per kU of enzyme, whereby the dynamic range over which the change in light absorbance occurs is increased.

10. The method of Claim 9 wherein the enzyme is glucose oxidase or cholesterol oxidase.

11. The method of Claim 10 wherein the alkali metal dichromate is sodium dichromate or potassium dichromate.

12. The method of Claim 11 wherein the enzyme is glucose oxidase and the alkali metal dichromate is potasium dichromate.

13. The method of Claim 9, wherein in the composition the soluble ferricyanide compound is reducible in the presence of the reaction between the enzyme and the analyte to produce a ferrocyanide compound, the composition further comprising a soluble ferric compound which reacts with a ferrocyanide compound to form a visually or optically detectable reaction product, and a buffer which does not prevent formation of the reaction product, wherein the compositiion has a pH of from about 3.0 to about 7.0.

14. The method of Claim 13 wherein the alkali metal dichromate is selected from the group consisting of potassium dichromate and sodium dichromate.

15. The method of Claim 14 wherein the enzyme is glucose oxidase or cholesterol oxidase.

16. The method of Claim 15 wherein the enzyme is glucose oxidase and the alkali metal dichromate is potasium dichromate.

17. Method for determining an analyte which reacts with the enzyme of the composition of Claim 4 in a liquid sample, the method comprising contacting the liquid sample with the composition of Claim 4 and determining formation of said reaction product as a measure of the analyte.

18. The method of Claim 17 wherein the liquid sample is a body fluid.

19. The method of claim 17 wherein the analyte is glucose or cholesterol.

20. An analytical element useful in determining an analyte in a sample, comprising
(a) a support carrier;
(b) a reagent layer applied to the support carrier, the reagent layer comprising the composition of Claims 1 or 4.

## Patentansprüche

1. Reagenzienzusammensetzung, die für die Verwendung bei Farbänderungsanalysen zur Bestimmung eines Analyten in einer flüssigen Probe geeignet ist und einen erweiterten dynamischen Bereich der Änderung des Lichtreflexionsvermögens und/oder der -extinktion aufweist, umfassend ein Enzym, das auf einen in der Probe vorhandenen Analyten einwirkt, eine lösliche Eisen(III)-cyanid-Vermittlerverbindung, die infolge der Einwirkung des Enzyms auf den Analyten reduziert wird, um eine Farbänderung im Reagens zu bewirken, die der Gegenwart oder Konzentration des Analyten entspricht, sowie etwa 0,9·10⁻⁴ bis etwa 17,5·10⁻⁴ Gramm pro kE Enzym eines Alkalimetalldichromats, das in der Reagenzienzusammensetzung löslich ist.

2. Reagenzienzusammensetzung nach Anspruch 1, wobei das Enzym Glucoseoxidase oder Cholesterinoxidase ist.

3. Reagenzienzusammensetzung nach Anspruch 1, wobei das Enzym Glucoseoxidase ist und das Alkalimetalldichromat Natriumdichromat oder Kaliumdichromat ist.

4. Reagenzienzusammensetzung nach Anspruch 1, wobei die lösliche Eisen(III)-cyanid-Verbindung in Gegenwart der Reaktion zwischen dem Enzym und dem Analyten unter Bildung einer Eisen(II)-cyanid-Verbindung reduzierbar ist, des weiteren umfassend eine lösliche Eisen(III)-Verbindung, die mit einer Eisen(II)-cyanid-Verbindung reagiert, um ein visuell oder optisch erfaßbares Reaktionsprodukt daraus zu bilden, welches die Lichtextinktion der Reagenzienzusammensetzung relativ zur Konzentration des Analyten verändert, sowie einen Puffer, der die Bildung des Reaktionsprodukts nicht verhindert, wobei die Zusammensetzung einen pH von etwa 3,0 bis etwa 7,0 aufweist.

5. Zusammensetzung nach Anspruch 4, wobei das Alkalimetalldichromat Natriumdichromat oder Kaliumdichromat ist.

6. Zusammensetzung nach Anspruch 5, wobei das Enzym Glucoseoxidase oder Cholesterinoxidase ist.

7. Zusammensetzung nach Anspruch 6, wobei das Enzym Glucoseoxidase ist und das Alkalimetalldichromat Kaliumdichromat ist.

8. Zusammensetzung nach den Ansprüchen 1 oder 4, die auf einen Träger aufbeschichtet ist.

9. Verfahren zur Verbesserung des dynamischen Bereichs der Lichtextinktion einer Zusammensetzung, die zur Bestimmung der Gegenwart oder der Konzentration eines Analyten in einer flüssigen Probe brauchbar ist, wobei die Zusammensetzung ein Enzym umfaßt, einen löslichen Eisen(III)-cyanid-Vermittler, der infolge einer Reaktion zwischen einem Analyten und dem Enzym reduzierbar ist, sowie einen Reaktionsteilnehmer, der mit der reduzierten Form des Vermittlers eine Änderung der Lichtextinktion der Zusammensetzung über einen Bereich bewirkt, umfassend die Zugabe eines Alkalimetalldichromats zu der Zusammensetzung in einer Konzentration von etwa 0,9·10⁻⁴ bis etwa 17,5·10⁻⁴ Gramm pro kE Enzym, wodurch sich der dynamische Bereich erweitert, über den die Änderung der Lichtextinktion auftritt.

10. Verfahren nach Anspruch 9, wobei das Enzym Glucoseoxidase oder Cholesterinoxidase ist.

11. Verfahren nach Anspruch 10, wobei das Alkalimetalldichromat Natriumdichromat oder Kaliumdichromat ist.

12. Verfahren nach Anspruch 11, wobei das Enzym Glucoseoxidase ist und das Alkalimetalldichromat Kaliumdichromat ist.

13. Verfahren nach Anspruch 9, wobei die lösliche Eisen-(III)-cyanid-Verbindung in der Zusammensetzung in Gegenwart der Reaktion zwischen dem Enzym und dem Analyten unter Bildung einer Eisen(II)-cyanid-Verbindung reduzierbar ist, wobei die Zusammensetzung des weiteren eine lösliche Eisen(III)-Verbindung umfaßt, die mit einer Eisen(II)-cyanid-Verbindung reagiert, um ein visuell oder optisch erfaßbares Reaktionsprodukt zu bilden, sowie einen Puffer, der die Bildung des Reaktionsprodukts nicht verhindert, wobei die Zusammensetzung einen pH von etwa 3,0 bis etwa 7,0 aufweist.

14. Verfahren nach Anspruch 13, wobei das Alkalimetalldichromat ausgewählt ist aus der Gruppe bestehend aus Kaliumdichromat und Natriumdichromat.

15. Verfahren nach Anspruch 14, wobei das Enzym Glucoseoxidase oder Cholesterinoxidase ist.

16. Verfahren nach Anspruch 15, wobei das Enzym Glucoseoxidase ist und das Alkalimetalldichromat Kaliumdichromat ist.

17. Verfahren zur Bestimmung eines Analyten, der mit dem Enzym der Zusammensetzung nach Anspruch 4 in einer flüssigen Probe reagiert, umfassend das Zusammenbringen der flüssigen Probe mit der Zusammensetzung nach Anspruch 4 und das Bestimmen der Bildung des Reaktionsprodukts als Maß für den Analyten.

18. Verfahren nach Anspruch 17, wobei die flüssige Probe eine Körperflüssigkeit ist.

19. Verfahren nach Anspruch 17, wobei der Analyt Glucose oder Cholesterin ist.

20. Analytisches Element, das zur Bestimmung eines Analyten in einer Probe brauchbar ist, umfassend
(a) einen Auflageträger
(b) eine auf den Auflageträger aufgebrachte Reagenzienschicht, wobei die Reagenzienschicht die Zusammensetzung nach Anspruch 1 oder 4 umfaßt.

## Revendications

1. Composition de réactif utilisable dans les analyses par changement de couleur pour la détection d'un analyte dans un échantillon liquide et ayant une plage dynamique plus grande de changement de réflectance et/ou absorbance de la lumière, comprenant une enzyme qui va agir sur l'analyte présent dans l'échantillon, un composé médiateur du type ferricyanure soluble qui va être réduit en réponse à l'action de l'enzyme sur l'analyte en effectuant un changement de couleur dans le réactif correspondant à la présence ou à la concentration de l'analyte et dans la fourchette allant d'environ 0,9 x 10⁻⁴ à environ 17,5 x 10⁻⁴ grammes par kU d'enzyme d'un bichromate de métal alcalin qui est soluble dans la composition de réactif.

2. Composition de réactif selon la revendication 1, dans laquelle l'enzyme est la glucose oxydase ou la cholestérol oxydase.

3. Composition de réactif selon la revendication 1, dans laquelle l'enzyme est la glucose oxydase et le bichromate de métal alcalin est le bichromate de sodium ou le bichromate de potassium.

4. Composition de réactif selon la revendication 1, dans laquelle le composé du type ferricyanure soluble est réductible en présence de la réaction entre l'enzyme et l'analyte en produisant un composé du type ferrocyanure, comprenant en outre un composé ferrique soluble qui réagit avec un composé du type ferrocyanure en formant un produit de la réaction entre eux visuellement ou optiquement détectable, lequel produit de réaction modifie l'absorbance de la lumière de la composition de réactif en rapport avec la concentration de l'analyte, et un tampon qui n'empêche pas la formation du produit de réaction, où la composition a un pH compris entre environ 3,0 et environ 7,0.

5. Composition selon la revendication 4, dans laquelle le bichromate de métal alcalin est le bichromate de sodium ou le bichromate de potassium.

6. Composition selon la revendication 5, dans laquelle l'enzyme est la glucose oxydase ou la cholestérol oxydase.

7. Composition selon la revendication 6, dans laquelle l'enzyme est la glucose oxydase et le bichromate de métal alcalin est le bichromate de potassium.

8. Composition selon les revendications 1 ou 4 étalée sur un support.

9. Procédé pour améliorer la plage dynamique d'absorbance de la lumière d'une composition utile pour la détermination de la présence ou de la concentration d'un analyte dans un échantillon liquide, la composition comprenant une enzyme, un médiateur du type ferricyanure soluble qui est réductible en réponse à une réaction entre un analyte et l'enzyme et un réactif qui agit avec la forme réduite du médiateur en modifiant l'absorbance de la lumière de la composition sur toute une plage, le procédé comprenant l'ajout d'un bichromate de métal alcalin à la composition à une concentration allant d'environ 0,9 x 10⁻⁴ à environ 17,5 x 10⁻⁴ grammes par kU d'enzyme, moyennant quoi la plage dynamique sur laquelle a lieu la modification de l'absorbance de la lumière est augmentée.

10. Procédé selon la revendication 9, dans lequel l'enzyme est la glucose oxydase ou la cholestérol oxydase.

11. Procédé selon la revendication 10, dans lequel le bichromate de métal alcalin est le bichromate de sodium ou le bichromate de potassium.

12. Procédé selon la revendication 11, dans lequel l'enzyme est la glucose oxydase et le bichromate de métal alcalin est le bichromate de potassium.

13. Procédé selon la revendication 9, dans lequel dans la composition, le composé du type ferricyanure soluble est réductible en présence de la réaction entre l'enzyme et l'analyte en produisant un composé du type ferrocyanure, la composition comprenant en outre un composé ferrique soluble qui réagit avec un composé du type ferrocyanure en formant un produit de réaction visuellement ou optiquement détectable, et un tampon qui n'empêche pas la formation du produit de réaction, où la composition a un pH compris entre environ 3,0 et environ 7,0.

14. Procédé selon la revendication 13, dans lequel le bichromate de métal alcalin est choisi dans le groupe constitué du bichromate de potassium et du bichromate de sodium.

15. Procédé selon la revendication 14, dans lequel l'enzyme est la glucose oxydase ou la cholestérol oxydase.

16. Procédé selon la revendication 15, dans lequel l'enzyme est la glucose oxydase et le bichromate de métal alcalin est le bichromate de potassium.

17. Procédé de détection d'un analyte qui réagit avec l'enzyme de la composition de la revendication 4 dans un échantillon liquide, le procédé comprenant la mise en contact de l'échantillon liquide avec la composition de la revendication 4 et la détection de la formation dudit produit de réaction en tant que mesure de l'analyte.

18. Procédé selon la revendication 17, dans lequel l'échantillon liquide est un fluide corporel.

19. Procédé selon la revendication 17, dans lequel l'analyte est le glucose ou le cholestérol.

20. Elément analytique utile dans la détection d'un analyte dans un échantillon, comprenant
(a) une matrice support;
(b) une couche de réactif appliquée sur la matrice support, la couche de réactif comprenant la composition des revendications 1 ou 4.
